(19) Europäisches Patentamt
European Patent Office
Office européen des brevets

(11) **EP 3 415 483 B1**

(12) **FASCICULE DE BREVET EUROPEEN**

(45) Date de publication et mention de la délivrance du brevet:
**17.11.2021 Bulletin 2021/46**

(21) Numéro de dépôt: **18305704.1**

(22) Date de dépôt: **11.06.2018**

(51) Int Cl.:
*C04B 35/45* (2006.01)    *C04B 35/63* (2006.01)
*C04B 35/632* (2006.01)    *B01J 23/72* (2006.01)
*B01J 37/00* (2006.01)    *B01J 35/00* (2006.01)
*C01B 3/16* (2006.01)    *B01D 15/08* (2006.01)
*B01D 53/04* (2006.01)    *B01D 53/48* (2006.01)
*C10L 3/10* (2006.01)    *C10G 25/00* (2006.01)

(54) **PROCÉDÉ DE PRÉPARATION DE SOLIDES À PARTIR D'UN MÉLANGE D'AU MOINS DEUX POUDRES DE MALACHITE**

HERSTELLUNGSVERFAHREN VON FESTSTOFFEN AUS EINER MISCHUNG VON MINDESTENS ZWEI MALACHITPULVERN

PROCESS FOR PREPARING SOLIDS FROM A MIXTURE OF AT LEAST TWO MALACHITE POWDERS

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **13.06.2017 FR 1755303**

(43) Date de publication de la demande:
**19.12.2018 Bulletin 2018/51**

(73) Titulaires:
• **IFP Energies nouvelles**
  **92852 Rueil-Malmaison Cedex (FR)**
• **AXENS**
  **92508 Rueil-Malmaison Cedex (FR)**

(72) Inventeurs:
• **BAZER-BACHI, Delphine**
  **30340 SAINT PRIVAT DES VIEUX (FR)**
• **CHICHE, David**
  **69007 LYON (FR)**
• **LOPEZ, Joseph**
  **30340 SAINT JULIEN LES ROSIERS (FR)**
• **SERRES, Thomas**
  **30980 LANGLADE (FR)**
• **FRISING, Tom**
  **92000 NANTERRE (FR)**
• **DUCREUX, Olivier**
  **78430 LOUVECIENNES (FR)**
• **EUZEN, Patrick**
  **75001 PARIS (FR)**

(74) Mandataire: **IFP Energies nouvelles**
**Département Propriété Industrielle**
**Rond Point de l'échangeur de Solaize**
**BP3**
**69360 Solaize (FR)**

(56) Documents cités:
EP-A1- 2 591 842        WO-A1-95/24962
US-A- 3 988 263        US-A- 4 582 819
US-A1- 2008 064 883

• Zhong Chun Chen ET AL: "Effect of particle packing on extrusion behavior of pastes", JOURNAL OF MATERIALS SCIENCE, septembre 2000 (2000-09), pages 5301-5307, XP055448673, DOI: 10.1023/A:1004834526344 Extrait de l'Internet: URL:https://rd.springer.com/content/pdf/10 .102 3/A:1004834526344.pdf [extrait le 2018-02-07]

## Description

### DOMAINE DE L'INVENTION

L'invention porte sur un procédé de préparation de solides à base de composés du cuivre, ainsi que sur l'utilisation de ces solides pour, entre autre, l'élimination de composés soufrés de charges gazeuses ou liquides tels que des gaz naturels, des biogaz, des gaz de synthèse, des gaz contenant du dioxyde de carbone $CO_2$, ou des hydrocarbures liquides.

Les solides préparés selon l'invention peuvent également être utilisés pour l'élimination du monoxyde de carbone CO, de composés contenant du mercure, ou de composés contenant de l'arsenic dans des charges gaz ou liquide, ainsi que pour la catalyse de la réaction de Dussan (réaction de gaz à l'eau ou « water gas shift » selon la terminologie anglo-saxonne).

### ART ANTERIEUR

Les composés du cuivre sont connus de l'état de l'art pour leur aptitude à réagir avec les composés soufrés. La mise en œuvre d'hydroxycarbonate de cuivre comme phase active présente un intérêt notamment car la réaction avec l'$H_2S$ semble être particulièrement rapide.

De nombreux documents abordent la désulfuration en présence d'oxyde de cuivre.

US 7837964 décrit un matériau pour la désulfuration pouvant comprendre jusqu'à 99,8% poids d'oxyde de cuivre. Le matériau est préparé par précipitation.

US 4582819 décrit un procédé de désulfuration d'hydrocarbures liquides à l'aide de solides préparés à partir d'hydroxycarbonate de cuivre et d'alumine. Les précurseurs ne sont pas peptisés. Le solide subit un traitement thermique, qui dégrade partiellement l'hydroxycarbonate de cuivre afin d'obtenir le CuO, à une température supérieure à 260°C.

US 2013/047850 décrit un procédé de purification de gaz de synthèse ($H_2$, CO) à l'aide de solides à base de CuO, élaborés selon un procédé permettant d'empêcher ou atténuer la réduction du CuO lors de la mise en oeuvre industrielle sur gaz de synthèse. Pour ce faire, les solides sont préparés à partir d'hydroxycarbonate de cuivre et d'un additif halogéné, par exemple NaCl, et d'une étape de calcination à une température comprise entre 280 et 500°C afin de décomposer entièrement le carbonate de cuivre en CuO.

Les documents US 6007706 et EP 243052 décrivent l'élimination de composés soufrés par la mise en oeuvre d'un solide comprenant au moins 70% poids d'un composé de cuivre (carbonate ou oxydes ou autre).

Cependant, aucun de ces documents n'aborde la problématique de la résistance mécanique des matériaux préparés.

Le document FR 2940967 décrit la préparation d'un solide à base de ZnO et son utilisation pour la désulfuration de charges liquides ou gazeuses, ledit solide présentant une excellente résistance mécanique et une capacité de stockage accrue. Le procédé de préparation comprend les étapes de mélange de poudres de ZnO, de peptisation et de calcination. Selon ce document, la peptisation basique du ZnO permet une dissolution partielle du ZnO en milieu basique, ce qui entraîne une diminution de la taille des particules, une densification du solide via une meilleur dispersion, et donc une augmentation de la résistance mécanique.

Cependant la malachite, qui a pour composition $Cu_2(OH)_2CO_3$, est un composé basique qui est soluble non dans un milieu basique, mais en milieu acide. La peptisation basique n'a donc pas d'effet de solubilisation qui pourrait aboutir à une diminution des tailles de particules et, in fine, à une amélioration de la résistance mécanique telle que décrite dans FR 2940967.

Le document WO 95/24962 décrit des masses de captation contenant au moins 75% poids de carbonate, hydroxycarbonate, ou hydroxyde de cuivre. L'objectif du texte est d'obtenir des solides présentant une densité de chargement d'au moins 0,9 kg/L, voire 1,2 kg/L, afin d'améliorer la capacité de captation volumique des solides (c'est-à-dire la quantité de soufre captée rapportée au volume de solide, non pas à sa masse). Ce document indique qu'il ne faut pas que la température de séchage/calcination excède 150°C, voire 115°C, afin de ne pas décomposer les composés du cuivre mis en oeuvre. Ceci permet en effet de compter le $CO_2$ et/ou l'eau contenu dans les solides sous forme de carbonate et d'hydroxyde, qui contribuent ainsi à l'augmentation de densité. Au-delà de 150°C, la décomposition du carbonate, hydroxycarbonate, ou hydroxyde de cuivre aboutit à une baisse de la densité de chargement des solides. De plus, ce document ne décrit pas d'étape de peptisation.

Cependant, les solides préparés uniquement à partir d'hydroxycarbonate de cuivre décrits dans l'art antérieur ont des propriétés de résistance mécanique particulièrement faibles, même après traitement thermique, ce qui rend leur mise en œuvre délicate, avec en particulier des problèmes d'attrition et de production de fines.

Or la demanderesse a découvert un procédé de préparation de solides à partir d'hydroxycarbonate de cuivre qui présentent à la fois des propriétés de résistance mécanique accrues et des capacités de captation de soufre effectives supérieures à celles des solides de l'art antérieur.

## DESCRIPTION SOMMAIRE DE L'INVENTION

L'invention concerne un procédé de préparation d'un solide selon la revendication 1.

Les adsorbants obtenus possèdent, outre une très bonne résistance mécanique, une densité et une porosité optimisées, permettant de maximiser leur capacité massique utile tout en réduisant le front de dispersion lors de la captation de soufre.

## DESCRIPTION DETAILLEE DE L'INVENTION

Dans la suite de l'exposé, on entend par solide préparé selon l'invention aussi bien, et de manière non limitative, adsorbant, que catalyseur, ou masse de captation en fonction des usages dudit solide.

Dans la suite de l'exposé, on utilise indifféremment les termes malachite, hydroxycarbonate de cuivre, et $Cu_2(OH)_2CO_3$.

Par perte au feu du solide, notée PAF, on entend la perte de poids en %poids d'un échantillon de solide soumis à calcination à 550°C pendant 2 h.

Par poudre, on entend un ensemble de particules.

Dans l'exposé qui suit, la distribution de taille d'une poudre ou de particules est mesurée par granulométrie à diffraction laser, basée sur la théorie de la diffraction de Mie (G. B. J. de Boer, C. de Weerd, D. Thoenes, H. W. J. Goossens, Part. Charact. 4 (1987) 14-19). La distribution de la granulométrie de ladite poudre ou desdites particules est représentée par le diamètre médian, noté $D_{50}$, défini comme étant le diamètre de la sphère équivalente tel que 50% en volume desdites particules ou des particules constituant ladite poudre a une taille inférieure audit diamètre.

Dans l'exposé qui suit, on entend par surface spécifique, la surface spécifique B.E.T. déterminée par adsorption d'azote conformément à la norme ASTM D 3663-78 établie à partir de la méthode BRUNAUER-EMMETT-TELLER décrite dans le périodique « The Journal of American Society", 60, 309, (1938).

### Étape a) de mélange

Conformément à l'invention, le procédé de préparation comprend une étape a) de mélange d'un ensemble comprenant au moins deux poudres de $Cu_2(OH)_2CO_3$ de granulométries différentes et au moins un liant.

Ledit ensemble comprend au moins deux poudres de $Cu_2(OH)_2CO_3$ de granulométries différentes et au moins un liant.

Ledit ensemble comprend de 0,1 à 99,9% poids, avantageusement de 2 à 99,9% poids, de manière préférée de 5 à 99,9% poids, de manière très préférée de 5 à 99% poids, préférentiellement de 5 à 90% poids et très préférentiellement de 5 à 85% poids d'une première poudre de malachite dont le $D_{50}$ est compris entre 1 et 15 μm, de manière préférée entre 1 et 10 μm, et de manière très préférée entre 4 et 9 μm, et de 99,9 à 0,1% poids, avantageusement de 98 à 0,1% poids, de manière préférée de 95 à 0,1% poids, de manière très préférée de 95 à 1% poids, préférentiellement de 95 à 10% poids et très préférentiellement de 95 à 15% poids d'une deuxième poudre de malachite dont le $D_{50}$ est compris entre 25 et 100 μm, de manière préférée entre 25 et 80 μm, préférentiellement entre 30 et 50 μm, le pourcentage poids étant exprimé par rapport au poids total des poudres de malachite. Une distribution bimodale améliore la résistance mécanique du solide final obtenu.

Le diamètre médian pondéré (noté Dm) dudit ensemble est compris entre 10 et 45 μm, de préférence entre 15 et 45 μm, les bornes étant incluses. Le diamètre médian pondéré dudit ensemble est calculé par la formule :

$$Dm = \sum_{1}^{n} x_n(Pn) \times D_{50}(Pn)$$

Avec $x_n(Pn)$ la fraction massique de la poudre de malachite Pn dans ledit ensemble, $D_{50}(Pn)$ le $D_{50}$ de la poudre de malachite Pn dudit ensemble.

De manière avantageuse, ledit ensemble est exempt de poudre d'oxyde de cuivre CuO. L'ensemble est avantageusement mélangé dans l'étape a) à sec, c'est-à-dire sans ajout de liquide. Ladite étape a) peut être mise en oeuvre par exemple dans un malaxeur ou dans tout autre type de mélangeur. Cette étape permet d'obtenir un mélange homogène des constituants pulvérulents.

### Sources de $Cu_2(OH)_2CO_3$

Les poudres de $Cu_2(OH)_2CO_3$ sont issues de toute source connue de l'Homme du métier.

*Liants*

L'ensemble mélangé dans l'étape a) comprend au moins un liant. Ledit liant permet la mise en forme dudit adsorbant tout en lui conférant une bonne résistance mécanique. Ledit liant est avantageusement sous forme de poudre.

Tout liant bien connu de l'Homme du métier peut être utilisé. En particulier, ledit liant peut être choisi de façon avantageuse, par exemple, parmi les argiles, tels que les minéraux de type kaolinite, les minéraux de type palygorskite, et les minéraux argileux de type smectite, tels que la montmorillonite ou la bentonite. Ledit liant peut également être choisi dans le groupe constitué par l'alumine, un précurseur d'alumine, qui est préférentiellement de la boehmite, la silice et leurs mélanges. Il est tout à fait possible de combiner l'utilisation de liants de différente nature, tel que par exemple un liant de type "alumine" et un liant de type "argile", ou encore deux argiles de nature différente. Selon un mode préféré de préparation du solide selon l'invention, le liant est une argile de type bentonite.

La quantité de liant mise en œuvre dans le procédé de préparation selon l'invention est telle que ledit liant représente moins de 50% poids du solide préparé (exprimée sur la base de la matière sèche totale, c'est-à-dire après perte au feu), et dépend de l'application ciblée.

Que ce soit pour les composés du cuivre ou le(s) liant(s), il est tout à fait envisageable de procéder à des mélanges de plusieurs sources de chaque composé.

Lorsque le solide préparé selon l'invention est utilisé pour la désulfuration de charge liquide ou gaz, la teneur en liant dudit solide est de préférence comprise entre 15% poids et 25% poids (exprimée sur la base de la matière sèche totale, soit après perte au feu).

**Étape b) de peptisation et de malaxage**

Conformément à l'invention, le procédé de préparation d'un adsorbant comprend une étape b) de mise en contact du mélange de l'étape a) avec une solution aqueuse et malaxage de la pâte obtenue.

Cette étape aboutissant à l'obtention d'une pâte, permet la dispersion des constituants, à savoir les poudres d'hydroxycarbonate de cuivre ainsi que le(s) liant(s), et une dissolution partielle des constituants.

Sous l'action de la solution aqueuse, les phénomènes de dispersion et de dissolution des particules d'hydroxycarbonate de cuivre et de liant opérant lors du malaxage par mise en contact des différents constituants sont favorisés. Sans vouloir s'attacher de manière restrictive à une quelconque théorie, il est toutefois possible d'émettre l'hypothèse qu'une meilleure dispersion tant de l'hydroxycarbonate de cuivre et des particules de liant(s) va dans le sens de l'amélioration de la résistance mécanique obtenue in fine par ledit procédé de préparation.

Ladite solution aqueuse contient avantageusement un agent peptisant acide ou basique.

Ledit agent peptisant acide peut être l'acide nitrique, l'acide chlorhydrique, ou tout autre acide connu de l'Homme du métier, par exemple un acide inorganique tel que l'acide fluorhydrique, l'acide bromhydrique, l'acide chlorhydrique, l'acide nitrique, nitreux, sulfonique, sulfurique, perchlorique, ou bien encore un acide organique mono ou di-carboxylique tel que l'acide acétique, propionique ou butanoique.

Dans un arrangement particulier du procédé selon l'invention, la peptisation est opérée en employant une solution aqueuse acide contenant de l'acide nitrique. Le ratio masse de $HNO_3$ / masse d'oxydes métalliques est compris entre 0,5 et 10% poids, de préférence compris entre 0,5 et 6%, et avantageusement compris entre 0,5 et 3%.

La masse d'oxydes métalliques est calculée selon la relation suivante :

$$masse\ oxydes = \frac{2 \cdot M_{CuO}}{M_{Cu_2(OH)_2CO_3}} \cdot m_{Cu_2(OH)_2CO_3}$$

Avec $m_{Cu2(OH)2CO3}$ la masse de poudres de malachite $Cu_2(OH)_2CO_3$ introduite lors de l'étape a), $M_{CuO}$ la masse molaire du CuO (= 80 g/mol), $M_{Cu2(OH)2CO3}$ la masse molaire de la malachite $Cu_2(OH)_2CO_3$ (= 222 g/mol).

Ledit agent peptisant basique peut être une base inorganique telle que la soude, la potasse, l'ammoniaque, ou bien encore une base organique telle qu'une amine, composé à ammonium quaternaire, choisie par exemple parmi les alkyl-éthanol amines ou les alkyl-amines éthoxylées.

Dans un arrangement particulier du procédé selon l'invention, la peptisation est opérée en employant une solution aqueuse contenant un agent peptisant basique, telle que de préférence un agent peptisant basique choisi dans le groupe constitué par la soude, la potasse, l'ammoniaque, l'hydroxyde de tétraéthylammonium (TEAOH), le carbonate d'ammonium et leurs mélanges. Le ratio masse d'agent peptisant basique / masse d'oxydes métalliques est compris entre 1 et 10% poids, de préférence compris entre 2 et 8%, et avantageusement compris entre 2 et 5%. La masse d'oxydes est calculée selon la relation précédente.

Dans un autre arrangement particulier du procédé selon l'invention, la peptisation est opérée en utilisant dans l'étape b) une solution aqueuse sans acide ni base ajoutés. Il a été observé de manière surprenante qu'une peptisation à l'eau non additivée de base ou d'acide permet d'obtenir un solide présentant des performances améliorées en adsorption, en particulier des composés soufrés. Dans cet arrangement particulier, la solution aqueuse de ladite étape b) est avantageusement de l'eau déionisée, par exemple à l'aide d'une résine échangeuse d'ions.

La quantité de solution aqueuse mise en oeuvre est ajustée de façon à obtenir, à l'issue de la peptisation et quelle que soit la variante mise en oeuvre, une pâte qui ne coule pas mais qui n'est pas non plus trop sèche afin de permettre l'extrusion lors de l'étape c) dans des conditions convenables de pression bien connues de l'Homme du métier et dépendantes de l'équipement d'extrusion utilisé.

La mise en contact des réactifs ($Cu_2(OH)_2CO_3$, liant(s), solution aqueuse) est réalisée par malaxage, en batch ou en continu.

Pour le malaxage en batch, les équipements tels que par exemple les malaxeurs équipés de bras en Z, à rouleau ou à cames sont connus de l'Homme du métier, mais tout autre équipement de malaxage peut également être utilisé.

Il est envisageable, au cours du malaxage de l'étape b) selon l'invention, d'incorporer un ou des adjuvants d'extrusion, permettant ainsi d'améliorer l'écoulement de la pâte dans la filière lors de l'extrusion. Ces adjuvants, bien connus de l'Homme du métier, peuvent par exemple être choisis parmi les acides aliphatiques mono-carboxyliques, les composés aromatiques alkylés, les sels d'acide sulphonique, les acides gras, la polyvinylpyridine, la polyvinylpyrrolidone, l'alcool polyvinylique, les dérivés cellulosiques.

Ces adjuvants sont généralement ajoutés à une teneur comprise entre 0,1% poids et 10% poids, de préférence comprise entre 0,2% poids et 8% poids, sur la base de la masse totale des constituants introduits dans le malaxeur.

La durée de malaxage est généralement comprise entre 5 et 60 min, de préférence entre 20 et 50 min. La vitesse de rotation des bras du malaxeur est comprise entre 10 et 75 tours/minute, de façon préférée entre 25 et 50 tours/minute.

## Étape c) d'extrusion

Conformément à l'invention, le procédé de préparation d'un adsorbant comprend une étape c) d'extrusion de la pâte malaxée dans l'étape b) à une pression comprise entre 3 et 25 MPa.

Ladite étape c) d'extrusion peut être mise en œuvre dans tout type d'extrudeuse, par exemple dans une extrudeuse piston, mono-vis ou bi-vis. La géométrie de la filière, qui confère leur forme aux extrudés, peut être choisie parmi les filières bien connues de l'Homme du métier. Ces filières peuvent être, par exemple, de forme cylindrique, trilobée, quadrilobée, cannelée ou à fentes.

Le diamètre des filières est fixé en fonction du diamètre des solides souhaités à l'issue de l'étape de calcination.

L'étape b) de mise en contact et de malaxage et l'étape c) d'extrusion peuvent avantageusement être mises en oeuvre dans un même équipement. Dans un exemple de cette mise en oeuvre, la pâte malaxée peut être extrudée directement en bout d'un malaxeur continu de type bi-vis. Dans un autre exemple de cette mise en oeuvre, un ou plusieurs malaxeurs batch sont reliés à une extrudeuse.

Les extrudés obtenus à l'issue de l'étape c) sont avantageusement séchés à une température comprise entre 70 et 160°C pendant une durée comprise entre 1 et 24 h avant d'être calcinés dans l'étape d). Ce séchage peut être avantageusement effectué sous air ou bien de préférence sous air humide. L'intérêt du séchage est d'évacuer de façon douce une partie des composés volatiles présents, la calcination directe du solide pouvant entrainer l'apparition de micro-fractures. Le séchage sous air humide permet d'évaporer lesdits composés volatiles plus lentement que le séchage sous air.

## Étape d) de calcination

Conformément à l'invention, le procédé de préparation d'un adsorbant comprend une étape d) de calcination des extrudés à une température comprise entre 140°C et 500°C et une durée comprise entre 10 min et 6 heures, sous un flux gazeux comprenant de l'oxygène.

Ladite étape d) de calcination est réalisée sous un flux gazeux comprenant de l'oxygène. Ledit flux gazeux peut être avantageusement de l'air, ou un mélange gazeux comprenant un gaz inerte (par exemple de l'azote) et de l'oxygène. Ledit flux gazeux comprend de préférence au moins 5% volume, préférentiellement au moins 10% volume d'oxygène. Ledit flux gazeux comprend également avantageusement de l'eau, et de manière préférée jusqu'à 3% volume d'eau.

Ladite étape d) de calcination est réalisée à une température comprise entre 140°C et 500°C, de préférence entre 200°C et 500°C, et de manière préférée entre 200°C et 350°C, pendant une durée comprise entre 10 min et 6 h, de préférence entre 10 min et 4 h, préférentiellement entre 10 min et 3 h, très préférentiellement entre 10 min et 2 h, et de manière très préférée entre 15 min et 1 h.

L'étape de calcination permet en particulier de convertir une fraction de malachite en oxyde de cuivre.

A l'issue de l'étape d) de calcination, les extrudés présentent un diamètre compris entre 1 et 10 mm, de manière préférée entre 1 et 5 mm, et de manière très préférée entre 1,5 et 3,5 mm.

Le solide selon l'invention, obtenu par extrusion, a une forme proche d'un bâtonnet cylindrique. Ces bâtonnets peuvent selon les besoins être introduits dans un équipement permettant d'arrondir leur surface, tel qu'un drageoir, ou tout autre équipement permettant leur sphéronisation.

Le pourcentage massique d'oxydes (teneur en CuO provenant de la décomposition de la malachite) contenus dans le solide après perte au feu peut être déterminé selon la relation suivante :

$$\% masse\ oxydes\ après\ PAF = \frac{\frac{2 \cdot M_{CuO}}{M_{Cu_2(OH)_2CO_3}} \cdot m_{Cu_2(OH)_2CO_3}}{m_{liant} + \frac{2 \cdot M_{CuO}}{M_{Cu_2(OH)_2CO_3}} \cdot m_{Cu_2(OH)_2CO_3}}$$

Avec $m_{liant}$ la masse de liant introduite lors de l'étape a), $m_{Cu2(OH)2CO3}$ la masse de poudres de malachite $Cu_2(OH)_2CO_3$ introduite lors de l'étape a), $M_{CuO}$ la masse molaire du CuO (= 80 g/mol), $M_{Cu2(OH)2CO3}$ la masse molaire de la malachite $Cu_2(OH)_2CO_3$ (= 222 g/mol).

Le solide préparé selon l'invention comprend au moins :

- 50 à 99% poids, de préférence 60 à 95% poids, de manière plus préférée 75 à 85% poids équivalent en masse de CuO mesuré après perte au feu à 550°C pendant 2 h, teneur déterminée selon la relation précédente.

- 1 à 50% poids, de préférence 5 à 40% poids, de manière plus préférée 15 à 25% poids d'un liant, le pourcentage masse étant mesuré après perte au feu.

Ces teneurs en % poids sont exprimées par rapport à la masse totale du solide préparé au moyen du procédé selon l'invention, et mesurées après décomposition des précurseurs à 550°C pendant 2 h.

## Propriétés du solide obtenu par le procédé selon l'invention

Les propriétés mécaniques sont déterminées par le test d'écrasement grain à grain (EGG) décrit par la méthode ASTM D 6175-3. Celle-ci consiste à mesurer la force de rupture de chaque particule d'un échantillon représentatif comprenant au moins 50 particules. Le résultat est pondéré par la longueur de l'extrudé. La valeur d'EGG est la moyenne des forces de rupture mesurées et ramenées à l'unité de longueur de l'extrudé (exprimée en daN.mm$^{-1}$) pour l'ensemble des particules de l'échantillon.

Dans le cas des solides préparés selon l'invention, la valeur d'EGG est supérieure à 0,7 daN.mm$^{-1}$ (décaNewton par millimètre de longueur d'extrudé), de préférence supérieure à 0,9 daN.mm$^{-1}$, et ce quelles que soient les teneurs en hydroxycarbonate de cuivre mises en œuvre.

De plus, les solides obtenus utilisés comme adsorbants présentent des performances de désulfuration améliorées vis-à-vis du traitement des gaz et des liquides contenant les composés soufrés, en particulier $H_2S$, mercaptans, COS et $CS_2$.

De manière surprenante, les solides préparés par le procédé selon l'invention, à partir d'un mélange spécifique de poudres de malachite, présentent une résistance mécanique satisfaisante, pour des coûts de production et notamment des coûts d'approvisionnement des poudres de malachite de départ limités et donc industriellement acceptables.

## Utilisation du solide obtenu par le procédé selon l'invention

L'invention a également pour objet l'utilisation du solide préparé par le procédé selon l'invention.

Le solide préparé selon l'invention peut être utilisé pour purifier des charges gazeuses telles que par exemple des hydrocarbures gazeux tels que des gaz naturels, des biogaz, des gaz contenant du dioxyde de carbone $CO_2$, ou des gaz de synthèse tels que ceux mis en œuvre dans les installations de cogénération, dans les unités de synthèse chimique telles que les unités de synthèse du méthanol ou de synthèse Fischer-Tropsch, ou des liquides tels que les hydrocarbures utilisés comme charges dans les unités de reformage catalytique, d'isomérisation, ou d'hydrogénation.

Le solide préparé selon l'invention est avantageusement utilisé pour purifier toute charge gazeuse ou liquide contenant entre autres, des composés soufrés tels que $H_2S$, COS et/ou $CS_2$, et/ou des mercaptans, à une pression comprise entre 0,1 et 25 MPa, de préférence entre 0,1 et 15 MPa, et une température comprise entre 0 et 450°C, de préférence entre 15 et 300°C, de manière préférée entre 15 et 250°C.

En particulier, le solide préparé selon l'invention peut être avantageusement utilisé pour purifier la charge d'une unité de synthèse Fischer-Tropsch, en le mettant en œuvre dans un réacteur opérant à une pression comprise entre 0,1 et 15 MPa, de préférence comprise entre 1,5 et 5,0 MPa, à une température comprise entre 0 et 400°C, de préférence entre 0 et 220°C, de préférence entre 15 et 180°C.

Ledit solide préparé selon l'invention peut également être utilisé afin d'éliminer certains hétéroéléments, tels que par exemple le phosphore ou ses composés tel que la phosphine $PH_3$, et/ou le chlore particulièrement sous sa forme HCl, présents dans des effluents liquides ou gazeux, de préférence à une pression comprise entre 0,1 et 25 MPa, de préférence entre 1 et 15 MPa, et une température comprise entre 0 et 200°C.

Ledit solide préparé selon l'invention peut également être utilisé afin d'éliminer les métaux lourds, tels que par exemple le mercure, et/ou l'arsenic ou ses composés tel que l'arsine $AsH_3$, présents dans des effluents liquides ou gazeux, de préférence à une pression comprise entre 0,1 et 25 MPa, de préférence entre 1 et 15 MPa, et une température comprise entre 0 et 200°C.

En effet, si la charge à purifier contient en plus des composés soufrés du mercure, le solide préparé au moyen du procédé selon l'invention permet également l'élimination du mercure présent dans la charge à traiter.

Le solide préparé selon l'invention peut avantageusement subir une étape de sulfuration avant sa mise en œuvre industrielle dans un procédé de captation de mercure, si la charge à traiter ne contient pas de composés soufrés.

Le solide préparé selon l'invention peut également être utilisé afin d'éliminer le monoxyde de carbone CO présent dans des effluents liquides ou gazeux, à une température comprise entre 0 et 200°C et une pression comprise entre 0,1 et 25 MPa.

Le solide préparé selon l'invention est utilisé en mettant en contact la charge gazeuse ou liquide à traiter avec ledit solide dans un réacteur, qui peut être un réacteur en lit fixe, un réacteur radial, ou bien encore un réacteur en lit fluidisé.

Le solide préparé selon l'invention peut être réduit sous atmosphère réductrice telle qu'un flux d'hydrogène, un gaz de synthèse puis utilisé comme catalyseur des réactions de Dussan, ou « Water Gas Shift » selon la terminologie anglo-saxonne, ou comme catalyseur de la synthèse de méthanol à partir de gaz de synthèse.

Des conditions typiques d'utilisation du solide comme catalyseur de la réaction de synthèse de méthanol sont une température comprise entre 100 et 500°C, de préférence entre 150 et 300°C, voire de préférence entre 220 et 280°C, et une pression comprise entre 0,1 et 25 MPa, de préférence entre 1 et 15 MPa, et de manière encore plus préférée entre 5 et 10 MPa.

Des conditions typiques d'utilisation du solide comme catalyseur de la réaction de Dussan sont une température comprise entre 100 et 500°C, de préférence entre 150 et 300°C, voire de préférence entre 180 et 250°C, et une pression comprise entre 0,1 et 25 MPa, de préférence entre 1 et 15 MPa, et de manière encore plus préférée entre 1,5 et 10 MPa.

## DESCRIPTION DES FIGURES

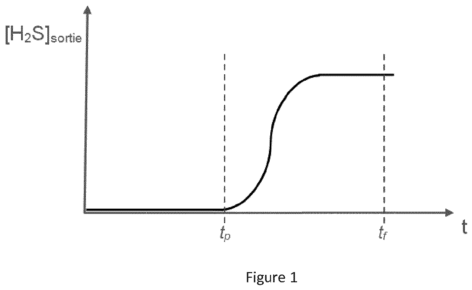

La Figure 1 est une représentation schématique d'une courbe de perçage pouvant être obtenue selon le protocole de mesure de la capacité de captation des impuretés par les solides, décrit ci-après. Sur la Figure 1, $t_p$ est le temps de perçage et $t_f$ le temps de fin de perçage.

## EXEMPLES

### Protocole de mesure de la capacité de captation des impuretés par les solides préparés

La capacité de captation en impuretés des solides préparés par le procédé selon l'invention est mesurée par un test de perçage.

Pour le test de détermination de la capacité de captation de $H_2S$, le test est réalisé à une température de 50°C, à une pression de 0,3 MPa, et avec une vitesse volumique horaire (VVH) de 1530 $h^{-1}$. Par vitesse volumique horaire, on entend le ratio du débit volumique de gaz mesuré à 0°C et 1 atm sur le volume de solide testé. Le gaz utilisé pour le test contient 0,9%vol. de $H_2S$ dans de l'azote. La teneur en $H_2S$ présente dans le gaz en sortie du réacteur contenant le solide est déterminée par chromatographie en phase gazeuse.

La capacité de captation d'une espèce *i* par le solide préparé par le procédé selon l'invention est déterminée en effectuant un bilan matière. La capacité de captation de l'espèce *i*, telle que définie au titre de la présente invention, correspond à la quantité d'espèce *i* accumulée par le solide avant perçage (c'est-à-dire au temps $t_p$ indiqué sur la Figure 1, qui représente schématiquement une courbe de perçage), celle-ci étant calculée à l'aide de la relation suivante :

$$q_i = M_i D_i^E \int_0^{t_p} \left(1 - \frac{C_i^S}{C_i^E}\right) dt$$

Avec :

$q_i$ : la masse de l'espèce $i$ captée par le solide (en g),

$D_i^E$ : le débit de l'espèce $i$ entrant (en mol.min$^{-1}$),

$M_i$ : la masse molaire de l'espèce $i$ (en g.mol$^{-1}$),

$C_i^E$ : la teneur en espèce $i$ du gaz en entrée,

$C_i^S$ : la teneur en espèce $i$ en sortie de réacteur,

$t_p$ : le temps nécessaire au perçage de l'espèce $i$ (en min) tel que représenté sur la Figure 1.

Sur la Figure 1, $t_p$ est le temps de perçage et $t_f$, le temps de fin de perçage.

La capacité de captation de l'espèce $i$ de le solide testé est donnée par la relation :

$$C_i = \frac{q_i}{m}$$

avec m la masse d'adsorbant mise en oeuvre lors du test.

**Exemple 1 : selon l'art antérieur**

Dans l'exemple 1, les solides de référence A1, A2, A3, A4 et A5 sont préparés selon le mode opératoire suivant :

a) mélange d'un ensemble comprenant une poudre de $Cu_2(OH)_2CO_3$ et un liant ;

b) mise en contact du mélange de l'étape a) avec une solution aqueuse (peptisation) et malaxage de la pâte obtenue dans un malaxeur muni de bras en Z, pendant 30 minutes avec une vitesse de ration des bras de 25 tours.minutes$^{-1}$ ;

c) extrusion de la pâte malaxée dans l'étape b) à l'aide d'une extrudeuse piston, en cylindres de diamètre 3 mm et de longueur 5 à 10 mm à une pression variable selon les solides ;

d) calcination des extrudés à une température variable selon les solides, conduite pendant une durée de 1 heure, sous flux d'air.

On utilise lors de l'étape a) une poudre de malachite P1 dont le $D_{50}$ est de 48 $\mu$m.

Une argile de type bentonite a été utilisée comme liant.

Les teneurs en CuO ou pourcentage massique d'oxydes (CuO provenant de la décomposition de la malachite) après perte au feu (550°C pendant 2 heures) sont de 80% poids pour les solides A1, A2, A3 et A4, et de 60% poids pour le solide A5 (le liant de type bentonite constituant le complément). Ces teneurs sont déterminées selon la relation suivante :

$$\%poids\ CuO\ après\ PAF = \frac{\frac{2 \cdot M_{CuO}}{M_{Cu_2(OH)_2CO_3}} \cdot m_{Cu_2(OH)_2CO_3}}{m_{liant} + \frac{2 \cdot M_{CuO}}{M_{Cu_2(OH)_2CO_3}} \cdot m_{Cu_2(OH)_2CO_3}}$$

Avec $m_{liant}$ la masse de liant introduite lors de l'étape a), $m_{Cu2(OH)2CO3}$ la masse de malachite $Cu_2(OH)_2CO_3$ introduite lors de l'étape a), $M_{CuO}$ la masse molaire du CuO (= 80 g/mol), $M_{Cu2(OH)2CO3}$ la masse molaire de la malachite $Cu_2(OH)_2CO_3$ (= 222 g/mol).

Pour les solides A1, A2 et A3, la quantité de base NaOH est de 4% masse rapportée à la quantité totale de $Cu_2(OH)_2CO_3$ introduite.

Pour les solides A4 et A5, de l'eau déionisée est utilisée comme solution aqueuse pour l'étape b) de malaxage.

Au cours de l'extrusion, la pression varie entre 50 et 150 bar selon la formulation utilisée.

Les formulations des solides A1, A2, A3, A4 et A5 sont données dans le tableau 1.

Tableau 1

| Désignation | %oxydes après PAF | Liant | Peptisation | Température de calcination (°C) / durée (h) | Test de captation de $H_2S$ : Soufre capté à tp (g S/g solide) | EGG (daN.mm$^{-1}$) |
|---|---|---|---|---|---|---|
| Solide A1 | 80% | Bentonite | 4% NaOH | 140°C / 1h | 0,22 | 0,5 |
| Solide A2 | 80% | Bentonite | 4% NaOH | 250°C / 1h | 0,25 | 0,5 |
| Solide A3 | 80% | Bentonite | 4% NaOH | 350°C / 1h | 0,24 | 0,5 |
| Solide A4 | 80% | Bentonite | eau | 250°C / 1h | 0,26 | 0,3 |
| Solide A5 | 60% | Bentonite | eau | 250°C / 1h | 0,16 | 0,6 |

La résistance mécanique des extrudés est déterminée par un essai mécanique de type écrasement grain à grain (EGG) tel que décrit précédemment.

La résistance mécanique des solides A1 à A5 est trop faible compte tenu des contraintes liées à une utilisation industrielle, Les valeurs d'EGG mesurées sont en effet inférieures à 0,7 daN.mm$^{-1}$, indépendamment de la température de calcination et de la présence ou non de soude lors de la peptisation lorsqu'une seule poudre présentant une seule distribution granulométrique est utilisée.

L'augmentation de la teneur en liant et la diminution de la teneur en malachite dans le solide A5 aboutit à une légère amélioration de la résistance mécanique qui reste toutefois insuffisante, au détriment de leur capacité de captation de soufre. Dans ce dernier cas, la capacité en soufre devient faible comparée à celle des solides selon l'invention.

## Exemple 2 : selon l'invention

Dans l'exemple 2, les solides référencés B1 à B4 selon l'invention sont préparés par malaxage-extrusion selon le mode opératoire suivant :

a) mélange d'un ensemble comprenant deux poudres de $Cu_2(OH)_2CO_3$ et un liant ;

b) mise en contact du mélange de l'étape a) avec une solution aqueuse (peptisation) et malaxage de la pâte obtenue dans un malaxeur muni de bras en Z, pendant 30 minutes avec une vitesse de rotation des bras de 25 tours.minutes$^{-1}$ ;

c) extrusion de la pâte malaxée dans l'étape b) à l'aide d'une extrudeuse piston, en cylindres de diamètre 3 mm et de longueur 5 à 10 mm à une pression variable selon les solides ;

d) calcination des extrudés à une température variable selon les exemples, conduite pendant une durée de 1 heure, sous flux d'air.

On utilise lors de l'étape a) deux poudres de malachite P1 et P2, présentant des granulométries différentes. Le $D_{50}$ de la poudre P1 est de 48 $\mu$m, et le $D_{50}$ de la poudre P2 est de 5 $\mu$m.

On exprime les quantités relatives de malachite P1 et P2 introduites à l'aide des ratio %P1 et %P2 définis par :

$$\% \: malachite \: P1 = \frac{m_{P1}}{m_{P1} + m_{P2}}$$

$$\% \: malachite \: P2 = \frac{m_{P2}}{m_{P1} + m_{P2}}$$

Avec $m_{P1}$ la masse de malachite P1 introduite lors de l'étape a), et $m_{P2}$ la masse de malachite P2 introduite lors de l'étape a),

Une argile de type bentonite a été utilisée comme liant.

La teneur en CuO provenant de la décomposition de la malachite après perte au feu (550°C pendant 2 heures) est de 80% poids pour les solides B1 à B4. Cette teneur est déterminée selon la relation suivante :

$$\%poids\ CuO\ après\ PAF = \frac{\dfrac{2 \cdot M_{CuO}}{M_{Cu_2(OH)_2CO_3}} \cdot m_{Cu_2(OH)_2CO_3}}{m_{liant} + \dfrac{2 \cdot M_{CuO}}{M_{Cu_2(OH)_2CO_3}} \cdot m_{Cu_2(OH)_2CO_3}}$$

Avec $m_{liant}$ la masse de liant introduite lors de l'étape a), $m_{Cu2(OH)2CO3}$ la masse des poudres de malachite $Cu_2(OH)_2CO_3$ introduites lors de l'étape a) (soit $m_{Cu2(OH)2CO3}$ = $m_{P1}$ + $m_{P2}$), $M_{CuO}$ la masse molaire du CuO (= 80 g/mol), $M_{Cu2(OH)2CO3}$ la masse molaire de la malachite $Cu_2(OH)_2CO_3$ (= 222 g/mol).

Pour les solides B1, B2, B3, B4, B5 et B6 de l'eau déionisée est utilisée comme solution aqueuse pour l'étape b) de malaxage.

Pour le solide B7, la quantité de base NaOH est de 4% masse rapportée à la quantité totale de $Cu_2(OH)_2CO_3$ introduite.

Au cours de l'extrusion, la pression varie entre 50 et 200 bar selon la formulation utilisée.

Les formulations des solides sont données dans le tableau 2.

Tableau 2

| Désignation | % P1 de la malachite totale | % P2 de la malachite totale | Dm (μm) | % oxydes après PAF | Liant | Peptisation | Température de calcination (°C) / durée (h) | Test de captation de $H_2S$ : Soufre capté à tp (g S/g solide) | EGG (daN.mm$^{-1}$) |
|---|---|---|---|---|---|---|---|---|---|
| Solide B1 | 87,5% | 12,5% | 42,6 | 80% | Bentonite | eau | 250°C / 1h | 0,25 | 0,8 |
| Solide B2 | 75% | 25% | 37,3 | 80% | Bentonite | eau | 250°C / 1h | 0,24 | 1,2 |
| Solide B3 | 50% | 50% | 26,5 | 80% | Bentonite | eau | 250°C / 1h | 0,26 | 1,2 |
| Solide B4 | 25% | 75% | 15,8 | 80% | Bentonite | eau | 250°C / 1h | 0,25 | 1,2 |
| Solide B5 | 87,5% | 12,5% | 42,6 | 80% | Bentonite | eau | 140°C / 1h | 0,23 | 0,7 |
| Solide B6 | 87,5% | 12,5% | 42,6 | 80% | Bentonite | eau | 350°C / 1h | 0,22 | 0,7 |
| Solide B7 | 87,5% | 12,5% | 42,6 | 80% | Bentonite | 4% NaOH | 250°C / 1h | 0,20 | 0,9 |

L'utilisation de deux poudres de $Cu_2(OH)_2CO_3$ de granulométrie différente dans le procédé de préparation selon l'invention permet, pour des Dm de mélange des poudres de départ compris entre 15 et 45 $\mu$m, d'obtenir des solides présentant des propriétés mécaniques satisfaisantes (EGG supérieure à 0,7 daN.mm$^{-1}$).

Par ailleurs, les solides présentent des capacités de sulfuration satisfaisantes, supérieures à 0,15 grammes de soufre/gramme de solide dans les conditions de test décrites dans le document.

**Exemple 3 : Comparatif**

Dans l'exemple 3, les solides C1 et C2 sont préparés par malaxage-extrusion selon le mode opératoire de l'Exemple 2.

Les deux poudres de malachite P1 et P2 utilisées pour préparer le solide C présentent les granulométries suivantes : le $D_{50}$ de la poudre P1 est de 50 $\mu$m et le $D_{50}$ de la poudre P2 est de 5 $\mu$m.

Le solide C est testé dans les mêmes conditions que celles des tests de l'Exemple 2.

Le Tableau 3 présente la formulation du solide C préparé et les résultats obtenus.

Tableau 3

| Désignation | % P1 de la malachite totale | % P2 de la malachite totale | Dm (µm) | % oxydes après PAF | Liant | Peptisation | Température de calcination (°C) / durée (h) | Test de captation de $H_2S$ : Soufre capté à tp (g S/g solide) | EGG (daN.mm$^{-1}$) |
|---|---|---|---|---|---|---|---|---|---|
| Solide C1 | 93,75% | 6,25% | 47,5 | 80% | Bentonite | eau | 250°C / 1h | 0,25 | 0,6 |
| Solide C2 | 96,87 | 3,13 | 48,7 | 80% | Bentonite | eau | 250°C / 1h | 0,25 | 0,6 |

Les valeurs d'EGG mesurées pour les solides C1 et C2 sont inférieures à 0,7 daN.mm$^{-1}$. La résistance mécanique des solides C1 et C2 est trop faible compte tenu des contraintes liées à une utilisation industrielle,

**Revendications**

1.  Procédé de préparation d'un solide comprenant les étapes de :

    a) mélange d'un ensemble comprenant au moins deux poudres de $Cu_2(OH)_2CO_3$ de granulométries différentes et au moins un liant, dans lequel :

    - ledit ensemble comprend de 0,1 à 99,9% poids d'une première poudre de malachite dont le $D_{50}$ est compris entre 1 et 15$\mu$m et de 99,9 à 0,1% poids d'une deuxième poudre de malachite dont le $D_{50}$ est compris entre 25 et 100 $\mu$m, le pourcentage poids étant exprimé par rapport au poids total des poudres de malachite,
    - le diamètre médian pondéré (Dm) dudit ensemble est compris entre 10 et 45 $\mu$m, ledit diamètre moyen pondéré étant calculé par la formule : $Dm = \sum_1^n x_n(Pn) \times D_{50}(Pn)$ avec : $x_n(Pn)$ la fraction massique de la poudre de malachite Pn dans ledit ensemble, $D_{50}(Pn)$ le $D_{50}$ de la poudre de malachite Pn dudit ensemble ;

    b) mise en contact du mélange de l'étape a) avec une solution aqueuse et malaxage de la pâte obtenue ;
    c) extrusion de la pâte malaxée dans l'étape b) à une pression comprise entre 3 et 25 MPa ;
    d) calcination des extrudés à une température comprise entre 140°C et 500°C et une durée comprise entre 10 min et 6 heures, sous un flux gazeux comprenant de l'oxygène

    le solide préparé comprenant 50 à 99% poids équivalent en masse de CuO et 1 à 50% poids de liant, les pourcentages masse étant mesurés après perte au feu et décomposition des précurseurs à 550°C pendant 2 h à 550°C pendant 2 h.

2.  Procédé selon l'une des revendications précédentes dans lequel ledit ensemble est mélangé dans l'étape a) à sec, c'est-à-dire sans ajout de liquide.

3.  Procédé selon l'une des revendications précédentes dans lequel ledit liant est choisi parmi les argiles, ou choisi dans le groupe constitué par l'alumine, un précurseur d'alumine, la silice et leurs mélanges.

4.  Procédé selon l'une des revendications précédentes dans lequel ladite solution aqueuse de ladite étape b) contient un agent peptisant acide ou basique.

5.  Procédé selon la revendication 4 dans lequel ladite solution aqueuse de ladite étape b) contient de l'acide nitrique, le ratio masse de $HNO_3$ / masse d'oxydes métalliques étant compris entre 0,5 et 10% poids.

6.  Procédé selon la revendication 4 dans lequel ledit agent peptisant basique est choisi dans le groupe constitué par la soude, la potasse, l'ammoniaque, l'hydroxyde de tétraéthylammonium (TEAOH), le carbonate d'ammonium et leurs mélanges, le ratio masse d'agent peptisant basique / masse d'oxydes métalliques étant compris entre 1 et 10% poids.

7.  Procédé selon l'une des revendications 1 à 3 dans lequel la solution aqueuse de ladite étape b) est une solution aqueuse sans acide ni base ajoutée.

8.  Procédé selon l'une des revendications précédentes dans lequel les extrudés obtenus à l'issue de l'étape c) sont séchés à une température comprise entre 70 et 160°C pendant une durée comprise entre 1 et 24 heures avant d'être calcinés dans l'étape d).

9.  Procédé selon l'une des revendications précédentes dans lequel l'étape d) de calcination est réalisée à une température comprise entre 200°C et 500°C.

10. Utilisation du solide préparé par le procédé selon l'une des revendications précédentes pour purifier des charges gazeuses ou des liquides.

**11.** Utilisation du solide préparé par le procédé selon l'une des revendications 1 à 9 comme catalyseur de la réaction de Dussan.

**12.** Utilisation du solide préparé par le procédé selon l'une des revendications 1 à 9 comme catalyseur de la réaction de synthèse de méthanol.

**Patentansprüche**

**1.** Verfahren zur Herstellung eines Feststoffs, das die folgenden Schritte umfasst:

a) Vermischen einer Gesamtheit, die mindestens zwei $Cu_2(OH)_2CO_3$-Pulver mit unterschiedlicher Korngrößenverteilung und mindestens ein Bindemittel umfasst, wobei

- die Gesamtheit 0,1 bis 99,9 Gewichts-% eines ersten Malachitpulvers, dessen $D_{50}$-Wert im Bereich von 1 bis 15 μm liegt, und 99,9 bis 0,1 Gewichts-% eines zweiten Malachitpulvers umfasst, dessen $D_{50}$-Wert im Bereich von 25 bis 100 μm liegt, wobei die Gewichtsprozentanteile unter Bezugnahme auf das Gesamtgewicht der Malachitpulver ausgedrückt sind,
- der gewichtete Medianwert des Durchmessers (Dm) bei der Gesamtheit im Bereich von 10 bis 45 μm liegt, wobei der gewichtete Medianwert nach der folgenden Formel berechnet wird: $Dm = \sum_1^n X_n(Pn) \times D_{50}(Pn)$

mit:
$x_n(Pn)$ als Massenanteil des Malachitpulvers Pn in der Gesamtheit, $D_{50}(Pn)$ als $D_{50}$-Wert des Malachitpulvers Pn der Gesamtheit;
b) Inkontaktbringen der Mischung des Schrittes a) mit einer wässrigen Lösung und Durchmischen der erhaltenen Paste;
c) Extrudieren der Paste, welche im Schritt b) durchmischt wurde, bei einem Druck im Bereich von 3 bis 25 MPa;
d) Brennen der Extrudatstücke bei einer Temperatur im Bereich von 140 °C bis 500 °C und über eine Dauer im Bereich von 10 min. bis 6 Std., unter einem gasförmigen Stoffstrom, der Sauerstoff umfasst

wobei der hergestellte Feststoff 50 bis 99 Gewichts-% an CuO-Massenäquivalenten und 1 bis 50 Gewichts-% an Bindemittel umfasst, wobei die Massenprozentanteile gemessen werden, nachdem die Brennschwindung erfolgt ist und sich die Vorstufen 2 Std. lang bei 550 °C 2 Std. lang bei 550 °C zersetzt haben.

**2.** Verfahren nach einem der vorhergehenden Ansprüche, wobei die Gesamtheit im Schritt a) trocken vermischt wird, das heißt, ohne Zusatz von Flüssigkeit.

**3.** Verfahren nach einem der vorhergehenden Ansprüche, wobei das Bindemittel aus den Tonen ausgewählt ist oder aus der Gruppe ausgewählt ist, welche aus Aluminiumoxid, einer Vorstufe von Aluminiumoxid, Siliciumdioxid und deren Mischungen besteht.

**4.** Verfahren nach einem der vorhergehenden Ansprüche, wobei die wässrige Lösung des Schrittes b) einen sauren oder basischen Peptisator enthält.

**5.** Verfahren nach Anspruch 4, wobei die wässrige Lösung des Schrittes b) Salpetersäure enthält, wobei das Verhältnis von Masse an $HNO_3$ / Masse an Metalloxiden im Bereich von 0,5 bis 10 Gewichts-% liegt.

**6.** Verfahren nach Anspruch 4, wobei der basische Peptisator aus der Gruppe ausgewählt ist, welche aus Soda, Pottasche, Ammoniumhydroxid, Tetraethylammoniumhydroxid (TEAOH), Ammoniumcarbonat und deren Mischungen besteht, wobei das Verhältnis von Masse an basischem Peptisator / Masse an Metalloxiden im Bereich von 1 bis 10 Gewichts-% liegt.

**7.** Verfahren nach einem der Ansprüche 1 bis 3, wobei es sich bei der wässrigen Lösung des Schrittes b) um eine wässrige Lösung handelt, der weder Säuren noch Basen zugesetzt wurden.

8. Verfahren nach einem der vorhergehenden Ansprüche, wobei die Extrudatstücke, wie sie nach Abschluss des Schrittes c) erhalten wurden, bei einer Temperatur im Bereich von 70 bis 160 °C über eine Dauer im Bereich von 1 bis 24 Stunden getrocknet werden, bevor sie im Schritt d) gebrannt werden.

9. Verfahren nach einem der vorhergehenden Ansprüche, wobei der Brennschritt d) bei einer Temperatur durchgeführt wird, die im Bereich von 200 °C bis 500 °C liegt.

10. Verwendung des Feststoffs, wie er mittels des Verfahrens nach einem der vorhergehenden Ansprüche hergestellt wurde, um gasförmige Chargen oder Flüssigkeiten aufzureinigen.

11. Verwendung des Feststoffs, wie er mittels des Verfahrens nach einem der Ansprüche 1 bis 9 hergestellt wurde, als Katalysator für die Wassergas-Shift-Reaktion.

12. Verwendung des Feststoffs, wie er mittels des Verfahrens nach einem der Ansprüche 1 bis 9 hergestellt wurde, als Katalysator für die Reaktion zur Methanolsynthese.

**Claims**

1. Process for preparing a solid comprising the stages of:

   a) mixing a set comprising at least two $Cu_2(OH)_2CO_3$ powders with different particle sizes and at least one binder, wherein:

   - said set comprises from 0.1% to 99.9% by weight of a first malachite powder, the $D_{50}$ of which is between 1 and 15 $\mu$m, and from 99.9% to 0.1% by weight of a second malachite powder, the $D_{50}$ of which is between 25 and 100 $\mu$m, the percentage by weight being expressed with respect to the total weight of the malachite powders,
   - the weighted medium diameter (Dm) of said set is between 10 and 45 $\mu$m, said weighted medium diameter being calculated by the formula:

   $$Dm = \sum_1^n x_n(Pn) \times D_{50}(Pn)$$

   with:

   $x_n(Pn)$ being the fraction by weight of the malachite powder Pn in said assembly,
   $D_{50}(Pn)$ being the $D_{50}$ of the malachite powder Pn of said set;

   b) bringing the mixture of stage a) into contact with an aqueous solution and kneading the paste obtained;
   c) extruding the paste kneaded in stage b) at a pressure of between 3 and 25 MPa;
   d) calcining the extrudates at a temperature of between 140°C and 500°C and for a period of time of between 10 min and 6 hours, under a gas stream comprising oxygen;

   wherein the prepared solid comprises from 50% to 99% by weight, equivalent by mass, of CuO and from 1% to 50% by weight of binder, the percentages by mass being measured after loss on ignition and decomposition of the precursors at 550°C over 2 h at 550°C over 2 h.

2. Process according to one of the preceding claims, in which said assembly is mixed in stage a) under dry conditions, that is to say without the addition of liquid.

3. Process according to either of the preceding claims, in which said binder is chosen from clays or chosen from the group consisting of alumina, an alumina precursor, silica and their mixtures.

4. Process according to one of the preceding claims, in which said aqueous solution of said stage b) contains an acidic or basic peptizing agent.

5. Process according to Claim 4, in which said aqueous solution of said stage b) contains nitric acid, the mass of

HNO$_3$/mass of metal oxides ratio being between 0.5% and 10% by weight.

6. Process according to Claim 4, in which said basic peptizing agent is chosen from the group consisting of sodium hydroxide, potassium hydroxide, ammonia, tetraethylammonium hydroxide (TEAOH), ammonium carbonate and their mixtures, the mass of basic peptizing agent/mass of metal oxides ratio being between 1% and 10% by weight.

7. Process according to one of Claims 1 to 3, in which the aqueous solution of said stage b) is an aqueous solution without added acid or base.

8. Process according to one of the preceding claims, in which the extrudates obtained resulting from stage c) are dried at a temperature of between 70 and 160°C for a period of time of between 1 and 24 hours before being calcined in stage d).

9. Process according to one of the preceding claims, in which the calcining stage d) is carried out at a temperature of between 200°C and 500°C.

10. Use of the solid prepared by the process according to one of the preceding claims for purifying gaseous feedstocks or liquids.

11. Use of the solid prepared by the process according to one of Claims 1 to 9 as catalyst of the Dussan reaction.

12. Use of the solid prepared by the process according to one of Claims 1 to 9 as catalyst of the reaction for the synthesis of methanol.

Figure 1

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 7837964 B
- US 4582819 A
- US 2013047850 A
- US 6007706 A

- EP 243052 A
- FR 2940967
- WO 9524962 A

**Littérature non-brevet citée dans la description**

- **G. B. J. DE BOER ; C. DE WEERD ; D. THOENES ; H. W. J. GOOSSENS.** *Part. Charact.,* 1987, vol. 4, 14-19

- *The Journal of American Society,* 1938, vol. 60, 309